# EUROPEAN PATENT SPECIFICATION

(11) **EP 2 903 551 B1**
(45) Date of publication and mention of the grant of the patent: **03.11.2021**
(21) Application number: 13785660.5
(22) Date of filing: 10.09.2013
(51) Int. Cl.: A61B 34/00, G02B 27/01, G06F 3/01, A61B 1/00

(54) **DIGITAL SYSTEM FOR SURGICAL VIDEO CAPTURING AND DISPLAY**
DIGITALES SYSTEM FÜR VIDEOERFASSUNG UND -ANZEIGE IN DER CHIRURGIE
SYSTÈME NUMÉRIQUE POUR LA CAPTURE ET L'AFFICHAGE D'UNE VIDÉO CHIRURGICALE

(30) Priority: 10.09.2012 IL 22186312
(43) Date of publication of application: 12.08.2015
(60) Divisional application: 20000134.5
(73) Proprietor: Elbit Systems Ltd., 31053 Haifa (IL)
(72) Inventor: SCHNEIDER, Ron, 3440503 Haifa (IL)
(74) Representative: Schuhmann, Albrecht
(86) International application number: PCT/IL2013/050764
(87) International publication number: WO 2014/037953

(56) References cited:
- WO-A1-2011/142165
- WO-A2-2009/094646
- JP-A- 2002 085 330
- US-A1- 2002 082 498
- US-A1- 2009 245 600
- W. Birkfellner ET AL: "A head-mounted operating binocular for augmented reality visualization in medicine - design and initial evaluation", IEEE TRANSACTIONS ON MEDICAL IMAGING., vol. 21, no. 8, 1 August 2002 (2002-08-01) , pages 991-997, XP055541269, US ISSN: 0278-0062, DOI: 10.1109/TMI.2002.803099
- Jonathan Cohen: "Comprehensive Atlas of High Resolution Endoscopy and Narrowband Imaging", , 15 April 2008 (2008-04-15), page 10, XP055698257, Retrieved from the Internet: URL:https://books.google.nl/books?id=5Qr-F hDahYcC&pg=PA24&lpg=PA24&dq=endoscope+digi tal+zoom&source=bl&ots=vsc4a6hfpJ&sig=ACfU 3U3r333N5241jDe3b63DfeVsrxGoWg&hl=en&sa=X& ved=2ahUKEwj534mpkNHpAhUSzaQKHQPvCZUQ6AEwD HoECAoQAQ#v=onepage&q=endoscope%20digital% 20zoom&f=false [retrieved on 2020-05-26]
- Anonymous: "Digital zoom - Wikipedia", , 25 August 2012 (2012-08-25), XP055698247, Retrieved from the Internet: URL:https://en.wikipedia.org/w/index.php?t itle=Digital_zoom&oldid=509149980 [retrieved on 2020-05-26]
- Anonymous: "Comparison of virtual reality headsets - Wikipedia", , 4 December 2020 (2020-12-04), XP055756853, wikipedia.org Retrieved from the Internet: URL:https://en.wikipedia.org/wiki/Comparis on_of_virtual_reality_headsets [retrieved on 2020-12-04]

## Description

### BACKGROUND

### 1. TECHNICAL FIELD

The present invention relates to the field of medical imaging, and more particularly, digital surgical capturing and display.

### 2. DISCUSSION OF RELATED ART

Currently available techniques for microscope surgical procedures utilize a classic optical microscope. The basic classical surgical microscope is constructed of high quality optical components, a zoom objective lens, eyepiece for user view and an XY motor. The user (or surgeon) sees the relevant object through direct optical channels. There are techniques to fold the light rays in the microscope to design a more comfortable and ergonomic structure for the user. However, because the microscopes are based on direct light rays channels, they are limited in their ability and flexibility to locate and shift the eyepiece. In addition, due to the classic direct view method of the microscope, with no digital means and sensors, the users do not benefit the digital domain advantages.

During prolonged use of the microscope (in particular, surgical operations), the user must position his head fixed to the microscope for a long period of time. The long time that the user holds his head in the microscope may cause neck pains.

Currently available techniques of endoscopic procedures (laparoscopy, colonoscopy and the like) include projecting the video on large monitor/s fixed in the operating room (OR). The endoscope is coupled to a digital camera. The camera video is projected on a large display. The entire staff, in particular the lead surgeon, must view the operation when their head is fixed to the monitor direction. The lack of ability to move their heads, view each other and their instruments causes the surgery to be more complicated, risky and increases the operation time (in some of the cases it may also cause an increase in the number of staff members). It is highly difficult for the surgeon supporting staff to assist since they do not see his direct actions and do not share his perspective.

U.S. Patent Application Publication No.: US 2009/0245600 A1 is directed to a digital zoom and panning system for digital video for use in minimally invasive robotic surgical systems. The system discloses a minimally invasive surgical device that includes an image acquisition device (endoscopic camera), an image buffer, a display device, a digital mapping and filtering device, and a user interface. The image buffer is coupled with the endoscopic camera, and the digital mapping and filtering device. The endoscopic camera is physically moved into tissue of a patient (i.e., a minimally invasive surgical site) and captures digital video images. The image buffer stores frames of the digital video images as source pixels. The user interface receives user input that includes a source rectangle to select source pixels within frames of the digital video images, a destination rectangle to select target pixels within the display device to display images, and a region of interest within the digital video images to display in the destination rectangle. The digital mapping and filtering device selectively maps and filters the source pixels in a region of interest from the image buffer into target pixels in a destination rectangle of the display device, according to the user input.

PCT International Publication No.: WO 2011/142165 A1 is directed to an operation input device and a manipulator system. The system includes a manipulator system that includes at least one manipulator, an operation input device having a display unit, an endoscope, an operation unit, a head-mounted unit, relative positions sensors, and a control unit. The at least one manipulator includes motors that change its respective position, orientation, and actuation status. The at least one manipulator and the endoscope are inserted inside the body of a patient. The head-mounted unit is mounted on the head of an operator. The operating unit operates a displayed object which is displayed on the display. The endoscope captures video images of the insides of the patient including a video image of the manipulator. The relative position sensors detect the relative position and the relative orientation of the head-mounted unit with respect to the operation unit. When the relative position and the relative orientation of the operating unit change with respect to the head-mounted unit, the control unit determines whether the change is due to a displacement of the head-mounted unit or a displacement of the operating unit. The control unit generates a control signal for moving the endoscope due to the displacement of the head-mounted unit, and generates control signals for the manipulators due to the displacement of the operating unit. The control unit actuates the display object that is displayed according to changes in the relative position and the relative orientation detected by the relative position sensors. The captured video images from the endoscope are displayed on the head-mounted unit for viewing by the operator.

Japanese Patent. Application No.: JP 2002-85330A is directed to a stereoscopic vision endoscopic apparatus for viewing an object facilitating depth perception by a user. The stereo vision endoscopic apparatus includes two optical systems (an optical system pair), a hard stereoscopic vision endoscope, two camera control units (CCU)s, a stereoscopic vision display, a monitor, a zoom control unit, polarized glasses, and a switch. The hard stereoscopic vision endoscope includes an elongated insertion part, and a base end part. The elongated insertion part includes two object optical systems exhibiting parallax for forming to-be-photographed images, and two relay optical systems for transmitting to-be-photographed images (for viewing by the left and right eyes of a user). The base end part includes two prisms, two eye pieces, two eyepiece optical systems, two variable-magnification optical systems, and two television (TV) cameras. Each of the variable-magnification optical systems includes drive mechanisms, which in turn include motors and gears. Each of the TV cameras includes a charged-coupled device (CCD) sensor that is connected to a respective eye piece. The to-be-photographed images are transmitted from the two object optical systems to the CCD sensors. The two prisms reflect an optical axis by 90 degrees. The TV cameras acquire images of the object. The CCUs receive the images from the TV cameras and signal-process them so as to output a video signal. The stereoscopic vision display displays the two observed images having a parallax. The zoom control unit includes motor drivers, which in turn control the motors. An edge part of image data from the CCUs is extracted and then compared (between right and left optical system image data) and based on the result of the comparison the zoom control unit controls the motor drivers. Via the motor drivers, the zoom control unit controls the variable-magnification optical systems such that the drive mechanisms move an optical axis direction back and forth and in so doing vary magnification, until the pair of to-be-photographed object images from the CCUs become substantially equal. Pressing the switch controls the drive mechanisms, effectively controlling the magnification. The monitor displays the two images in alteration or simultaneously. The polarized glasses enable viewing of the object with a three-dimensional (3-D) effect.

### BRIEF SUMMARY

The invention is defined in the appended claims. One embodiment of this disclosure provides a system which integrates data from surgical visualization instruments, such as microscopes, laparoscopes, data from existing medical imaging instruments, such as MRI and CT scans, patient's medical history, and current vital signs available in the operating room (OR). The new systems also detach the user from any fixed instruments, making the environment more ergonomic. The system can display 3D roadmap and reference data coming from instruments in the OR. The data can come from imaging instruments capturing real time 3D data. Another method is to receive data from different angles of capturing, and the system will generate the 3D data for display.

Another embodiment of this disclosure provides a digital system which replaces the classic optical surgical microscope, while maintaining the optical resolution and field of view (FOV) of the microscope. Two digital cameras are used to capture images of the operated area from slightly different angles, later to be displayed to each eye, providing 3D imaging. Another method for capturing images from different angles may be utilizing one camera with a special optical assembly. The cameras capture a large FOV, corresponding to the largest FOV captured by surgical microscopes. The image is displayed on dual display head mounted device. The user gaze direction (user's eye line of sight) is measured by head and eye trackers. The captured images and gaze direction are routed to the system's computer. According to the gaze direction the computer crops from the camera images the relevant region-of-interest (ROI) and feeds them into a specially designed display system. It should be understood that the ROI can be of any shape and tailored for the purposes of the application.

In another embodiment the ROI may be selected using an input coming from keyboard, mouse, touchscreen, and interfaces. Apart from 3D imaging, the dual-display system provides high resolution video, comparable to that of the human eye, at the user's current area of interest. The display can be designed to present high resolution in its entire field of view or can be designed to maintain low resolution in the peripheral FOV for situational awareness. According to one embodiment, the ROI can be derived by tracking a point of a tool (e.g., scalpel) held by a surgeon. This way, the ROI is being updated in real time as the operation progresses.

Yet another embodiment of the present invention provides digital system for applications that do not require an optical microscope. The applications can be related (but not only) to imaging in the area of orthopedic surgery, gynecological surgery, otolaryngology, neurosurgery, oncologic surgery, pediatric, oral and maxillofacial, plastic surgery, catheterization, laparoscopy and colonoscopy. The video source is captured by the computer, along with the head tracker. In this system, the computer crops the relevant ROI (if any) and transmits the image to the display. The display can be single or dual-eye type, allowing 2D or 3D, according to the nature of the application and video source. See-through display allows the user peripheral vision. The ROI as referred herein in this application may include not only the location of the ROI within the image but also meta data relating to viewing angle, type of objects to be viewed and further data that may be presented within the ROI such as messages and alerts.

Consonant with some embodiments of the present invention, the aforementioned systems may be provided with elements such as: The processor and video sources can support multiple users having their own set of gaze direction tracker and display. The video can also be forwarded to different locations for consultation and teaching purposes; The video can be recorded for future analysis and presentation; The image of the main video source may be space-stabilized, allowing the positioning of other virtual instruments at directions convenient to the user, giving patients history, current vital signs, past test results, CT scans, and the like. The head tracker may be used to control steerable instruments in the operating room.

### BRIEF DESCRIPTION OF THE DRAWINGS

For a better understanding of embodiments of the invention and to show how the same may be carried into effect, reference will now be made, purely by way of example, to the accompanying drawings in which like numerals designate corresponding elements or sections throughout.

In the accompanying drawings:

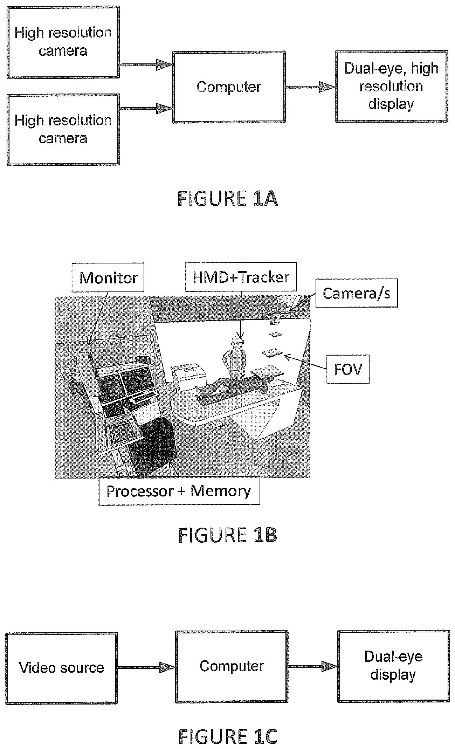

Figure 1A is a schematic block diagram illustrating one aspect according to some embodiments of the present invention;
Figure 1B is a perspective view illustrating another aspect according to some embodiments of the present invention;
Figure 1C is a schematic block diagram illustrating another aspect according to some embodiments of the present invention;
Figure 1D is a perspective view illustrating another aspect according to some embodiments of the present invention;
Figure 2A is a schematic block diagram illustrating yet another aspect according to some embodiments of the present invention;
Figure 2B is a perspective view illustrating yet another aspect according to some embodiments of the present invention;
Figure 2C is an optical diagram of yet another aspect according to some embodiments of the present invention;
Figure 2D shows graphs illustrating yet another aspect according to some embodiments of the present invention;
Figure 2E shows perspective views illustrating yet another aspect according to some embodiments of the present invention;
Figure 2F shows images illustrating yet another aspect according to some embodiments of the present invention; and
Figure 2G shows images illustrating yet another aspect according to some embodiments of the present invention.

### DETAILED DESCRIPTION

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

The present invention, in embodiments thereof, includes several main building blocks as follows: high resolution camera or cameras having a large FOV; a head mounted display capable of projecting the desired resolution and FOV to the user; a head tracker, and an eye tracker. Embodiments of the present invention provide an angular resolution that is comparable or better than that of the human eye, and capable of object magnification similar to that of surgical microscopes (magnification up to ~30-40).

Additionally, some other elements may also be used as follow: a light source to illuminate the scene and object, processors, and interface devices. The system interfaces the users, operating-room equipment, and necessary infrastructure, such as communication.

According to some embodiments of the present invention, two systems for digital microscopy are provided herein. A third system, mainly for endoscope systems, is also described herein, but does not fall within the scope of the invention. Many of the elements of the systems are common to both systems, and are described below, followed by a detailed description of the differences between the systems.

The proposed systems may use part or all of the following common elements. One such element is a video camera. The proposed systems use cameras to capture live images of the operated area. The image is displayed to the head mounted display (HMD) or monitor. The camera or cameras can be: Replacement to the microscope - the camera has a lens capable of focusing on objects at a distance of tens of centimeters. The camera or cameras can be add-on to the microscope - the add-on can be by using mechanical adapter to fit it in a dedicated port (surgical microscopes utilize beam splitter to split the light to the user's eyepiece and to additional ports to be used by cameras, other eyepieces and the like).

The user's gaze direction is measured by head and/or eye trackers. The camera captured images and gaze direction are routed to the system's processor. The processor crops relevant ROI from the high resolution camera images. The ROI images resolution are fit to the display resolution. The ROI location (or coordinates) is calculated according to the gaze direction.

The processor has input data ports for important medical data. The data can be patient history data, monitoring data or any external medical device.

The processor performs image processing on the captured images. The processing can be: Image correction, like fixed pattern noise corrections, color corrections, distortion corrections, histogram enhancement and the like; Adding markers on relevant image fixtures, tracking objects, emphasizing objects and the like; Overlaying monitoring data or patient history data; Fusing the video with other imaging device like CT, MRI etc.

The processor transmits the captured ROI images into display ports. The display device can be one of the following: High quality and high resolution color HMD. The HMD can be dual- eye display for stereo video or single-eye display for mono video. A part from the HMD the display can be any monitor or projecting device displaying the image. The monitor can be 3D monitor or standard 2D monitor. All the data or part of it can be saved on the system memory. The control on the system activation and important functions can be done using foot pedal, key-pad control box or voice activation.

Several types of systems are described herein. The differences between the systems are detailed below.

System I is a system for digital surgical microscopy. In this system the camera or cameras capture wide FOV area, larger than the display FOV. The camera resolution is very high so it is capable of capturing the entire FOV with all the necessary details in it (for large magnification purpose). After first adjustments, the system is of no need with mechanical motions mechanisms, like XY movement or optical zoom. Because the camera captures the entire FOV of interest, data magnification or areas of interest are extracted digitally.

System II is a system for digital surgical microscopy. The camera or cameras capture wide field of view (FOV) area, larger than the display FOV. The camera resolution is very high but may need mechanical motions, like XY movement or optical zoom to capture extended FOV.

System III is a system for digital surgical endoscopy and does not form part of the invention. In this type of system video is received from external imaging source like laparoscopy, colonoscopy, neurosurgery, MRI, CT etc. Consonant with embodiments of the present invention, the camera used in the systems maintains the standard required from high end surgical microscopes which support the human high optical performance standards. That is, sharp 3D image while maintaining situational awareness for the FOV provided by the microscope. The camera maintains these benefits and provides the user with increased functionality and situational awareness, while detaching him from the microscope. The camera main features are: 3D camera with ultra high resolution; 3D camera with resolution to cover the full FOV of the microscope; and Camera with ROI slaved to the HMD.

In one case the camera can be added on to surgical microscopes. Using adaptors and optics the camera will be connected to the microscopes. In another case, the camera will be with an objective lens (or photographic lens) which will be used to replace the entire surgical microscope. The camera will image the object of interest and transmit the image to the processor unit and to the display (monitor or HMD). It is crucial to maintain small time latency from the grabbing of the image to the image display. The small latency is crucial so the user will not suffer from delay between movements in the object plane till the image is displayed.

In some embodiments, two cameras are used to capture the images of the left and right eye (provided by the microscope) for stereo imaging. The cameras are positioned in two different angles to provide the display with images from different angles that will eventually create 3D imaging. This configuration may be implemented in parallel to the existing eyepieces or by replacing them (by either upgrading existing microscopes or replacing them with new instruments).

In other embodiments, one or more cameras can be used to capture images from different positions. The capturing can be done for fix location of the camera/cameras or by changing the cameras position to different locations. At every location the cameras captures the image and the processor saves the images in the memory. The images captured from the camera/cameras can be used to generate 3D model of the object. It can be made at real-time for fixed cameras or non-real-time for moving cameras. Example use for this embodiment can be creation of 3D model of the head and brain for neurosurgery. The camera/cameras are moved to different locations around the subject head. The images are saved in the memory. The processor uses the images to generate 3D model of the head/brain. Example algorithm for such 3D model can be implemented using radon transform. This model can be used during the surgery to help the surgeon navigate or improve the 3D orientation. The 3D model can be displayed to surgeon in 3D display, head mounted display, 2D display as 3D model. The 3D database can also be used for non-display purpose. The processor can perform different algorithms on the database, like image correlation, tracking and the like.

According to some embodiments, where a plurality of displays and a plurality of users of several types are present, the computer processor may be configured to apply a plurality of predefined image processing or video processing algorithms to at the at least one selected ROI and present outputs of the algorithms over the plurality of displays. In this manner, each user may select his or her own ROI and receive a tailored processed image by applying the image processing algorithms or any other operation applied to the user-specific ROI and display. By the aforementioned embodiments, the computer processor becomes means for managing the systems resources in terms of image sensors, input devices, ROIs to be selected and the various displays present on the scene.

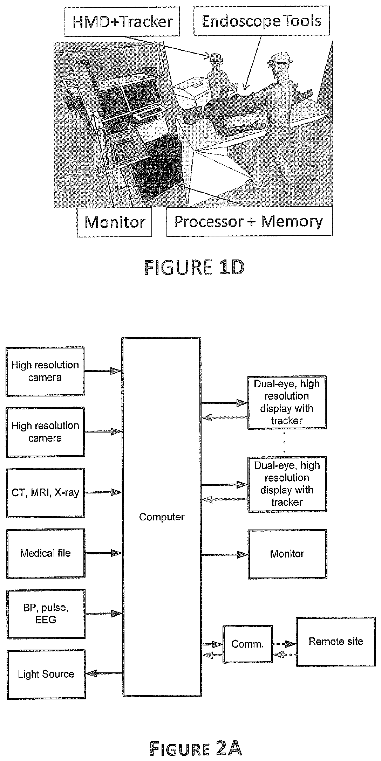

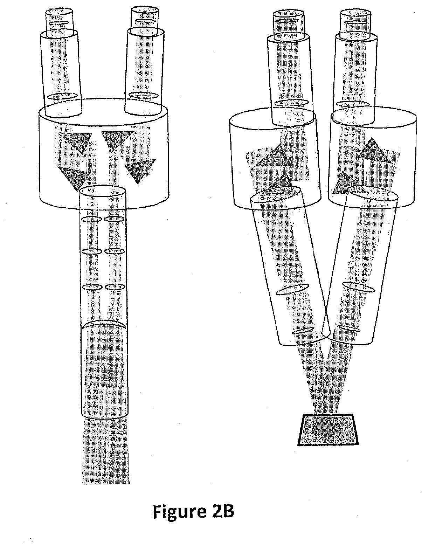

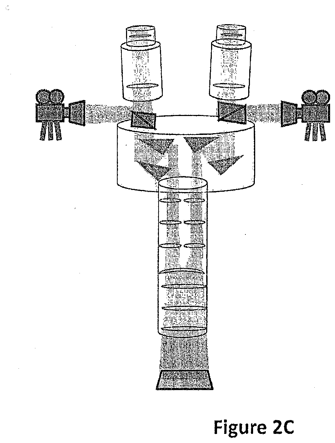

As shown in Figure 2B, some of the surgical microscopes employ a common main objective (CMO) design. A major advantage of this design is the ability to incorporate prisms into the optical path which allow the diversion of some of the light without impacting substantially the quality of the image. Cameras can be placed at some of the predesigned beam splitting location to capture the image of each of the optical paths, one for each eye, as shown in Figure 2C.

Another method of designing the surgical microscopes is by using the Greenough design, which gives several advantages. This design allows the location of the microscope at a large distance from the patient. The large distance is important for lowering the risk of patient contamination. The design of the microscope is simpler, makes the instrument production cheaper, and supports higher numerical apertures and greater depths of field. The use of digital cameras enables optical distortions correction, such as the keystone effect, and introduces accessories independent of the optical design, much like the CMO design. In the case where the cameras are add-on to microscopes with robotic (motorized) arm, the motor can be enslaved to the head and eye movements - a benefit in some fields of brain surgery.

Referring now to the surgical microscope camera, in the first system concept decried above (system I), the camera captures the full FOV of the microscope. The camera resolution is high enough so the system angular resolution is comparable to that of the human eye. The cameras can be assembled as stand-alone or attached to the microscopes using the CMO or Greenough designs. From the captured images, ROIs are cropped and sent to the display unit. The ROIs coordinates depend on the LOS of the head and/or the eye. The ROI coordinates can be also extracted from joystick, keyboard, foot pedestal, voice activation and any other input device. Capturing the full FOV at maximum resolution makes it feasible to display the user with a human-eye-like resolution for its area of interest. This working method allows several benefits to the user: providing the user with a superb image; zooming in to the image digitally; digital XY motion in the image; providing a different user with an image of its area of interest simultaneously to the primary user; recording the entire FOV of the microscope for debriefing and later analysis; and sharing the image observed by the user for the purpose of teaching or receiving assistance.

The cropping of the ROI out of the full frame can be carried out in several methods. The first method involves grabbing the full image out of the sensor. In this method the ROI is digitally cropped from the full image after the grabbing. The full image or only the ROI can be stored in the processor memory. The second method involves grabbing only the ROI from the sensor and not the full frame. The grabbing can be made by addressing only the relevant pixels in the image array. In the first two methods, the processor performs image resize on the ROI so it will fit the resolution of the display unit. A third method, which does not form part of the present invention, involves grabbing and under-sampling of the ROI from the sensor. In this method the digital image sensors are configured to under-sample for adjusting the spatial resolution of the video sequence to the spatial resolution of the electronic display. An example of under-sampling is the sampling of every second pixel in the array instead of every pixel.

In the first method, using an ROI digitally cropped from the full image, the ROI is a powerful tool for improving surgery. When the full high resolution is grabbed to the processor memory important data can be used for different applications. In the simplest way, the entire data can be saved for late debrief. During the operation, each user can select different ROI, according to required coordinates and magnification, without disturbing the other users. This is important, as an example, for complicated surgeries, were more than one surgeon are operating at the same time. Not only surgeons can use the different ROI's, but students, nurses or other staff members, users which are not in the same operating room etc. The ROI's can be used for multiple users, were the other user is the processor. For example, the surgeon can mark an area of interest, where the processor tracks that there is no blood flow or other defined event. The user can mark different ROI's with bookmarking. In this case the user can return to the bookmarked ROI in a frame time. The time transition from ROI to ROI is frame time. It includes magnifications change (from large to small values), XY change or emergency change.

The second system concept described above (system II) is to enslave the LOS of a narrow FOV camera, having the same angular resolution as the above camera, to the head and/or eye tracker. This allows the capturing of smaller resolution images, decreasing the amount of data to be handled by the system, while still maintaining the necessary resolution. A fast bi-axial steering mirror scans the image obtained through a large FOV optics onto a small FOV camera. The position of the mirror is enslaved to the user's head and eye movements, replacing the cropped area of the previous solution. Additional method for shift the LOS of the camera is by using pan and tilt mechanism of some elements of the system.

In another embodiment there is a combination of the first and second concepts. In this concept the camera has a moderate resolution with scanning possibilities. In addition there is internal ROI to display on the HMD or monitor.

The HMD may be in the form of a display device, worn on the head, which has a small display optic in front of one or each eye. The HMD can produce 2 dimensional images or 3 dimensional images (stereoscopic display). In the context of the present invention, the HMD is referred as a tool for displaying the video, symbols, or any type of displayable information. To achieve high quality video, the HMD can be implemented displaying color image, 3D (for every eye), high resolution and high FOV. However, one or more of the mentioned qualities might not be implemented.

A see-through HMD may be used in some embodiments, thus allowing a video to be superimposed on a real-world view. For example, superimposing real-world view with video can be done by projecting the video through a partially reflective surface and viewing the real world directly. Combining real-world view with video can also be done electronically by accepting video from a camera capturing the real world and mixing it electronically with video from the system.

Usually HMD images are focused to infinity. Focus to infinity provides the viewer relaxed effort for the eye and less exhausting. However, when using see-through HMD, and the real world objects are not located at infinity (for example, closed room), the focus of the HMD needs to be set to the objects distance. In general, HMDs can utilize a dynamic focus mechanism, setting the projected image focus distance to the viewer eye focus distance (or to the distance of the object that the viewer sees).

An HMD can utilize a head tracker. The head tracker can be used to locate the user head location in space and by so calculating the viewer LOS (line of Sight). Knowledge of the line of sight of the viewer is a powerful tool allowing different unique functions and applications. For example, in specific, a see-through HMD with head tracker can be implemented to fix a virtual video in space (set fixed relative to the real world), to display data in any real world location. In a general manner, it can be used to fix any data or video to a specific coordinate in space.

Additionally, the user LOS may be used for different applications. For example, the LOS can be used as a controller for microscope motors (for translator motion or zoom) instead of a joystick.

Another example is the change of an ROI (region of interest) in the displayed image. The change in ROI can be its coordinate in the image or its size (digital zoom).

The HMD utilizes the head tracker so it is possible to present symbols, information, images and the like in the field of regard of the user. The FOV can be defined around a specific line of sight (for example directly to the object under operation). The field of regard can be defined around the FOV.

According to some embodiments, the computer processor may be configured to project the video sequences over the electronic display in a first and a second regions, wherein an angular resolution of the second region is higher than a threshold such as 0.0005 radians and wherein the angular resolution of the first region is lower than the aforementioned threshold of 0.0005 radians, and wherein the first region has a field of view of at least of for example 2° and is substantially centered around the LOS of the user.

The HMD can utilize eye tracker. The eye tracker can be used to locate the exact eye LOS relative to the HMD device. Knowledge of the eye LOS can be used to implement smart displays were the projected image is depended on the observer eye LOS. In general, most of the functions mentioned regarding the head tracker can be implemented using the eye tracker. In combination with the head tracker, the HMD can be a very friendly tool to visualize data fixed in space and to be used as controller.

In order to meet the present invention, the HMD must produce high quality video. The HMD quality of video must be acceptable for the user, when compared to the image viewed through regular (not digital) microscope eyepiece. To achieve high quality video from the HMD several critical parameters must be implemented - high angular resolution, wide FOV, high color quality and high dynamic range. In the scope of this invention we refer only to the first two parameters - high angular resolution and wide FOV.

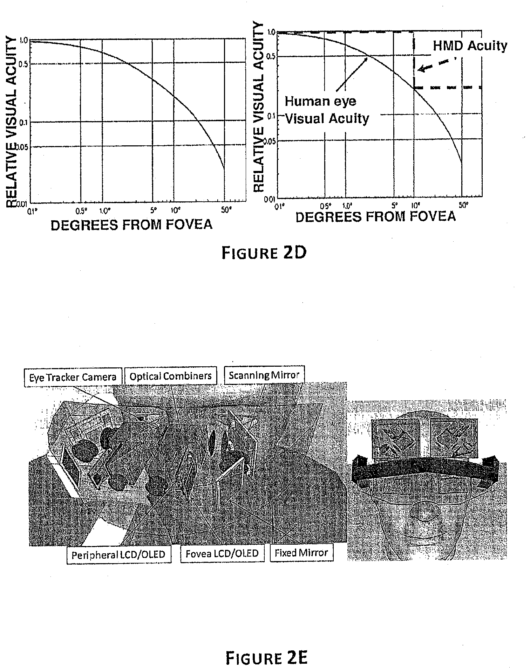

The user of the HMD is the human viewer. To achieve high quality display, the HMD optical parameters need to be good enough to satisfy the human vision. The FOV of the human vision is very large. Yet, the human angular resolution (or visual acuity) is depended on the location of the image on the retina. There is a small area (small FOV) of the retina with improved visual acuity. This area is called the fovea, and covers about 2 degrees of visual FOV. When drawing away from the fovea the visual acuity degrades monotonically (see Figure 2D, left image). The human viewer will not notice improvement in resolution or have the ability to see more details using HMD with smaller angular resolution than the human vision (see Figure 2D, right image). So, in general, a high quality HMD will have angular resolution similar or better than the human vision.

Referring to surgical microscope HMD, two exemplary and non-limiting implementations of HMD which will produce a high quality image, and which will have small enough angular resolution, so the human viewer will not feel the effect of observing digital image are described herein:
The first implementation of the HMD is by producing constant angular resolution as a function of the FOV. In general this approach utilizes a high resolution display as part of the HMD.

The second implementation of the HMD is by producing varying angular resolution as a function of the HMD FOV. The angular resolution must be better than the human vision, so in this implementation, small angular resolution must be projected in the FOV of the fovea line of sight. In the peripheral FOV (around the fovea) the angular resolution may be larger than in the fovea area (Figure 2D). The HMD has a mechanism to shift the projected image (to the viewer) according to eye motion (using eye tracker). The direction of the projected image in this case follows the viewer LOS, so it is depended to the eye motion; the viewer sees an image in front of him.

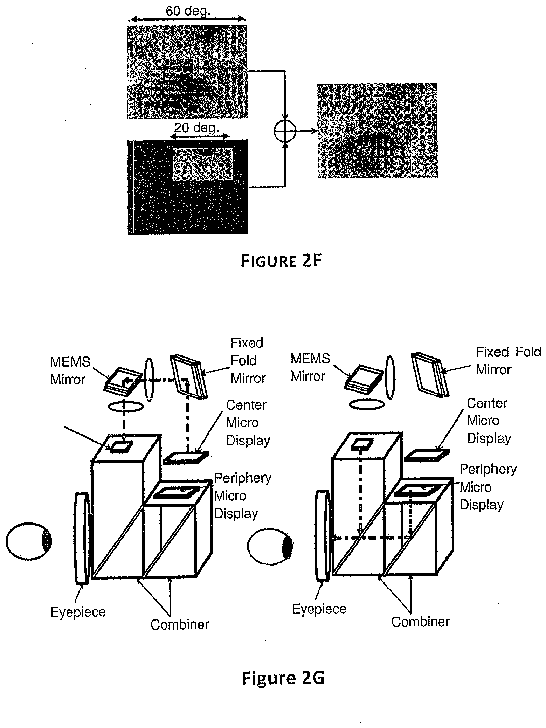

Example for a design of the second implementation of the HMD (producing varying angular resolution) is illustrated in Figure 2E. In the example there are two micro-displays for each eye. One micro-display is used for the peripheral image. The second micro-display is being used for the fovea image. The second micro-display is projected at a narrow FOV relative to the second micro-display. The two projected images (of the two micro displays) are combined using an optical combiner. With an eyepiece the viewer can see the combined image (Figure 2F). To achieve better transition between the two superimposed images some smoothing can be done in the stitching area. For example, in the edges of the fovea image, the resolution can be degraded monolithically (using image processing smearing) down to the resolution of the peripheral image. This will cause super-imposing the images to look more natural.

The image seen by the viewer must be with angular resolution better than the human visual acuity. To achieve that with the second implementation of the HMD, the fovea image micro-display (center micro-display) must be moved according to the LOS of the viewer. One way to achieve it is by using mirror (MEMS for example) to shift the center micro-display image on the eyepiece focal plane. The rotating mirror (2 Axis) can be shifted using an eye tracker - enslaving the mirror to the viewer LOS. An example implementation is shown in Figure 2G. The optical components described in Figure 2F can be assembled directly in front of the eye or as a see-through HMD.

The HMD projected image can be achieved using micro-display (LCD, OLED, LCOS), DMD (Digital Micro-mirror Device), DLP (Digital Light Processing), scanning mirrors or any other method. In general the HMD can be implemented as a see-through device where the viewer can see the displayed image and the scenery behind it. The HMD can be implemented as a wireless or wired device communicating and receiving images from an external source.

Referring now to the head and eye trackers, some of the uses of the trackers may include: controlling the movement of robotic arm; controlling the movement of the ROI in the high resolution display; controlling the image Zoom; stabilizing the image in space; controlling the see-through brightness. Head and eye trackers are powerful tools. Their main role in the system application is to provide the LOS of the user's area of interest. In general, the head tracker provides the LOS for the displayed image FOV, while the eye tracker provides the LOS for the high resolution FOV. The LOS provided by the head trackers allows to crop the relevant ROI from the ultra-high resolution image or to direct the fast steering mirror to the appropriate angle. This image is then decimated before being displayed by the peripheral display.

The head tracker LOS enables the HMD to create space-stabilized objects. These objects include the image from the microscope and any further data that needs to be available to the user, such as medical history, previously obtained CT scans, current patient vital statistics, as measured by other O.R. instruments, etc. These may be placed at user-defined spatial locations. For example, looking at 45° to the left, the user can see the patient's last CT scan, looking forward gives the microscope image, looking down allows to see the patient through the display, and looking up provides the time display. For many users, the ability to provide a stable image is crucial for the prevention of nausea.

As mentioned above, the head tracker may also be used to perform other tasks, via the user interface, or as a source for directing microscope movements or other medical equipment.

Reference is now made to a wide FOV situation awareness (SA) camera. The SA camera may not be part of the normal optical channel of the microscope. Optical microscopes do not provide the user the tools for SA. When the user eyes are fixed to the microscope eye piece the vision is limited to the limited FOV of the scope. Events that occur outside the microscope are hidden from the user. In addition there is no ability to maneuver the microscope LOS to different object based on seeing.

Embodiments of the present invention may provide further increase of the peripheral FOV with extended SA. The increase of FOV is provided using lower angular resolution camera or cameras. The additional cameras may be located outside the microscope, in a perimeter close to the microscope objectives. The wide FOV cameras images may be fused (or superimposed) with the images obtained through the microscope optics and provide further peripheral FOV. Another technique for displaying the wide FOV images is in a different window in the HMD field of regard, or to present the wide FOV images overlapping part of the microscope image.

Reference is now made to voice activation of the system. Controlling the system may be implemented in several ways, via dedicated control panels, specially designed levers and buttons to be used by the user using his/her feet, etc. Of special interest is the voice activation technology, which allows hands-free control, leaving the user's hands free to perform the additional tasks, while providing a flexible and a rich interface. Voice recognition allows the identification of pre-programmed key-words, reserved for specific purposes, to be used by any user, and to allow users to program their own key-words, independent of a language or dictionary. For example, the oral command "markers on" may add pre-defined markers onto the displayed image. A Spanish-speaking user may supplement the English commands with its native language commands by programming them himself.

Voice activation can be used in conjunction with the head and eye trackers to construct elaborate yet intuitive user interface. For example, the "zoom on" oral command may enable a head movement dependent image zoom. Moving the head forward zooms into the image, while moving it backwards zooms out. The "zoom off' command turns off this feature, allowing for normal operation.

Referring now to the main processor and memory unit, the main processor unit is used primarily to: interface with the camera, the HMD, the head tracker, the eye tracker, the video monitors, the voice activation hardware, any controller panels and the microscope; running different real time software applications; running video editing software applications; playing the video from the memory unit; interfacing other systems like MRI, CT and the like. The memory unit is used primarily to record the video or images from the cameras and running real time software applications.

Referring now to the main processor unit interfaces, the processor interface to the camera and includes camera gain control, exposure control and any other control setting the camera working point. In addition the processor sets the video frame rate and ROI to capture from the camera. The processor unit captures the video from the camera and if the user selects to record the data, pushes the video to the memory unit. The processor interface to the head tracker includes calculation of the LOS of the head. The head LOS can be calculated for 3 coordinates (azimuth, elevation and rotation) or six coordinates (also location in space) for more accurate applications. The processor interface to the eye tracker includes calculation of the LOS of the eye. The processor interface to the HMD includes HMD brightness control, contrast control and any other control setting the HMD displayed image. According to the eye and head tracker inputs, the processor pushes the HMD the relevant images, data and symbols. For complicated displays involving superposition of two micro displays (Figure 2F) the processor defines the images to push to the different micro displays. The processor may control or transfer relevant data for hardware in the HMD like the scan mirrors, head tracker, eye tracker camera or any other hardware.

The following are exemplary main processor unit real time applications. Head tracker applications may include: Controlling the microscope motors or ROI for translator movement and for optical or digital zoom. For example, when the user moves the head to different directions, microscope motors follows the head movement. In this way, for example, the viewer can divert the microscope to the desired object. The control of the optical zoom can be by moving the head toward and backward, setting the zoom in and zoom out function.

Another application is changing the brightness of the see-through HMD projected display. For example, the user can define that when looking in a specific direction the projected image is bright, and when moving the head to different location the image turns darker to enable higher appearance of the real world. According to the head location, the displayed image can be video, different symbols or different information.

Another embodiment is changing the see-through transparency as a function of the user head location. When the user's head is in one direction the transparency is low, so the projected image contrast is high. When the head moves to another direction, the transparency is high, so the user can have situation awareness through the HMD. Combination of the display brightness reduction can be used also, to improve the contrast of the background image. In general, the transparency change can be made on part of the see-through display or all of it. Transparency may be adjusted according to user manual selection and/or other ambient thresholds.

The transparency change can be made using passive or active coating/pads on the see-through display. These coating/pads are placed on a display element which is in the user line of sight. In general, it can be placed on visor, combiner, waveguide or any other optical element used for the see-trough display. The passive coating can decrease the see-through display transmission by using UV/NIR illumination on it. The active coating decrease transmission when electrically activated.

Eye tracker applications may include: For complicated displays involving superposition of two micro displays (Figure 2F) the eye tracker defines the location of the smaller angular resolution image (determines the image location on the eye piece focal plane). Shifting the entire projected display LOS.

According to the LOS of the viewer the see-through HMD can focus the projected image according to the real world object distance. With additional mechanism (like a camera) and the LOS of the viewer the HMD can detected the distance to the object and focus accordingly. This focus mechanism will prevent fatigues to the eye (due to changes in focus between the display and the real world). Applications that were described using the head tracker can be used also using eye tracker up to the extent of the human vision FOV.

The system and image processing applications may include: The processor generates different markers of interest features on the projected imaged. In the case of surgery the markers can be based to specify features to address. The processor generates important OR (operating room) equipment data and patient history information to present on the HMD projected display. The data can be presented on the video displayed or in the field of regard. Markers can be pushed from automatic data management or personal.

The above systems may utilize a light source. The most common approach is a light source designed to illuminate from the cameras optical line of sight. In this way the lens is built in such a way that the light from the source is directed through mirrors or beam splitters to illuminate through the lens. One more common approach is to illuminate from a singular point at some location near the lens. A third approach may be to use more than one light source. The multiple sources can be the ones described in the first two approaches. In addition a ring of light sources can be designed around the lens. The distribution of light sources produces uniform light with less shadowing in the image.

Once using the light sources it is possible to utilize the digital domain to control camera and the light source. For the camera it is common to have algorithms like AGC (automatic gain control) and AEC (automatic exposure control) to calculate and set the camera working point. The gain and exposure are usually calculated to produce image with maximum dynamic range and minimum saturations. With a single or multiple light sources an ALC (automatic light control) may be used. Based on the image the algorithm calculates the light levels of the single or multiple light sources. The controllable light sources facing the scenes are dynamically adjusted based on a dynamic analysis of the image histogram of the video sequences. The level of the lights are adjusted to optimize the contrasts, dynamic range and to minimize specular reflections from objects.

For some applications and procedures it is important to capture images in specific and limited spectral bands. For example capturing images in the NIR (near infra read) spectral band can enhance blood vessels. The light sources may be designed to radiate different spectral bands. One method to achieve the different spectrums is by using different light sources. For example the use of different types of LEDs, in different colors or in the NIR. A second method to achieve different spectrums is by using optical filters, like band pass filters in front of the light sources. One source can be covered by one type of filter, while a different source will be covered by a second type of filter.

Instead of designing the light sources for different spectrums, or in addition, the camera can be designed to capture images in different spectral bands. The camera optics may be designed so in some location in the optical path an optical filter is inserted. The mechanism of the optical filter can be designed to be fixed permanently or to support removal and insertion of the filter. The mechanism is not limited to one filter. For example a filter wheel or a light prism can be used to support multiple types of filters for different spectral bands. While performing the procedure, the user can set the spectral band by setting the corresponding optical filter.

The above systems may utilize a projector illuminating the imaged object. The projector can illuminate in the visible spectrum or in the near-infrared spectrum. The visual illumination can provide some visual guidance to the users. For example, to mark areas of interest, mark places to perform surgery cuts, direction symbols etc. Illuminating in the near-infrared can be used for structured light applications. The structured light is used to produce 3D data base of the objects. The projector can be of any type - DMD, Pico MEMS projector, LCD, CRT etc.

Another embodiment of this disclosure is guidance for the user. The guidance can be done by creating a path for the surgeon tools. The path is created using the 3D database created from images received from the cameras. Based on these images the system tracks the tool and guides the surgeon to the designated direction. The path can be generated automatically, manually or by a combination of both. The system can generate warning when the surgeon does not move the tool in right direction or gets close to sensitive areas.

In the above description, an embodiment is an implementation of the invention or of unclaimed other concepts of this disclosure. The various appearances of "one embodiment", "an embodiment" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment. Embodiments of the invention may include features from different embodiments disclosed above, and embodiments may incorporate elements from other embodiments disclosed above, as long as the result falls within the scope of the appended claims. The disclosure of elements of the invention in the context of a specific embodiment is not to be taken as limiting their used in the specific embodiment alone.

Furthermore, it is to be understood that the invention can be carried out or practiced in various ways and that the invention can be implemented in embodiments other than the ones outlined in the description above.

The invention is not limited to those diagrams or to the corresponding descriptions. For example, flow need not move through each illustrated box or state, or in exactly the same order as illustrated and described.

Meanings of technical and scientific terms used herein are to be commonly understood as by one of ordinary skill in the art to which the invention belongs, unless otherwise defined.

While the invention has been described with respect to a limited number of embodiments, these should not be construed as limitations on the scope of the invention, but rather as exemplifications of some of the preferred embodiments. Other possible variations, modifications, and applications are also within the scope of the invention, as long as they fall within the scope of the appended claims.

## Claims

1. A digital surgical microscopy system, comprising:
a camera configured to capture images of an operated area;
a head-mounted display (HMD) configured to display magnifications of said operated area to a user, by displaying region of interest (ROI) images cropped from said images;
a memory unit; and
a processing device coupled with said HMD, said camera, and said memory unit, said processing device configured to receive an input indicating said ROI, and configured to produce said ROI images by either one of:
grabbing a full image out of an image sensor of said camera, and digitally cropping said ROI from said full image; or
grabbing said ROI out of said image sensor and not said full image;
wherein said memory unit is configured to store said full image or said ROI;
wherein said digital surgical microscopy system is a non-endoscopy system.

2. The digital surgical microscopy system according to claim 1, further comprising a tracker configured to track a user line-of-sight (LOS) of said user, said digital surgical microscopy system further configured to change a ROI location or a ROI size, according to said LOS.

3. The digital surgical microscopy system according to claim 2, wherein said tracker is either one of an eye tracker, and a head tracker.

4. The digital surgical microscopy system according claim 1, wherein said processing device is configured to apply at least one predefined image processing or video processing, algorithm to said ROI images.

5. The digital surgical microscopy system according to claim 1, wherein said processing device is configured to add markers onto a displayed image by said HMD.

6. The digital surgical microscopy system according to claim 1, wherein said HMD is a see-through HMD whose transparency is adjustable.

7. The digital surgical microscopy system according to claim 6, wherein said transparency on part of said see-through HMD or all of said see-through HMD is adjustable.

8. The digital surgical microscopy system according to claim 1, wherein said grabbing involves either one of: grabbing from said image sensor in full resolution, and grabbing from said image sensor by under-sampling.

9. The digital surgical microscopy system according to claim 1, wherein said image sensor has a pixel resolution that is higher than a pixel resolution of said HMD.

10. The digital surgical microscopy system according to claim 1, wherein said processor performs image resize on said ROI to fit to the resolution of said HMD.

11. A non-endoscopic method in digital surgical microscopy for displaying magnifications of an operated area on a head-mounted display (HMD), the method comprising:
capturing, by a camera, images of an entire field of view (FOV) of interest in said operated area of a patient for digital magnification of said operated area, from a distance to said patient;
receiving an input indicating a region of interest (ROI) of said operated area;
producing ROI images that are cropped from said images by either one of:
grabbing a full image out of an image sensor, and digitally cropping said ROI from said full image; or
grabbing said ROI out of said image sensor, not from said full image; and
displaying said ROI images.

## Patentansprüche

1. Digitales chirurgisches Mikroskopiesystem, umfassend:
eine Kamera, die konfiguriert ist, um Bilder eines operierten Bereichs aufzunehmen;
ein Head-Mounted-Display (HMD), das konfiguriert ist, um Vergrößerungen des operierten Bereichs für einen Benutzer anzuzeigen, indem es Bilder einer Interessenregion (ROI), die aus den Bildern zugeschnitten ist, anzeigt;
eine Speichereinheit; und
eine Verarbeitungsvorrichtung, die mit dem HMD, der Kamera und der Speichereinheit gekoppelt ist, wobei die Verarbeitungsvorrichtung konfiguriert ist, um eine Eingabe, welche die ROI angibt, zu empfangen, und konfiguriert ist, um die ROI-Bilder durch eines zu erzeugen von:
Erfassen eines Vollbildes aus einem Bildsensor der Kamera und digitales Zuschneiden der ROI aus dem Vollbild; oder
Erfassen der ROI aus dem Bildsensor und nicht des Vollbildes;
wobei die Speichereinheit konfiguriert ist, um das Vollbild oder die ROI zu speichern;
wobei das digitale chirurgische Mikroskopiesystem kein Endoskopiesystem ist.

2. Digitales chirurgisches Mikroskopiesystem nach Anspruch 1, ferner umfassend eine Verfolgungsvorrichtung, die konfiguriert ist, um eine Benutzer-Sichtlinie (LOS) des Benutzers zu verfolgen, wobei das digitale chirurgische Mikroskopiesystem ferner konfiguriert ist, um eine ROI-Stelle oder eine ROI-Größe gemäß der LOS zu ändern.

3. Digitales chirurgisches Mikroskopiesystem nach Anspruch 2, wobei die Verfolgungsvorrichtung entweder eine Augenverfolgungsvorrichtung oder eine Kopfverfolgungsvorrichtung ist.

4. Digitales chirurgisches Mikroskopiesystem nach Anspruch 1, wobei die Verarbeitungsvorrichtung konfiguriert ist, um mindestens einen vordefinierten Bildverarbeitungs- oder Videoverarbeitungsalgorithmus auf die ROI-Bilder anzuwenden.

5. Digitales chirurgisches Mikroskopiesystem nach Anspruch 1, wobei die Verarbeitungsvorrichtung konfiguriert ist, um Markierungen auf einem von dem HMD angezeigten Bild hinzuzufügen.

6. Digitales chirurgisches Mikroskopiesystem nach Anspruch 1, wobei das HMD ist ein Durchsicht-HMD ist, dessen Transparenz einstellbar ist.

7. Digitales chirurgisches Mikroskopiesystem nach Anspruch 6, wobei die Transparenz auf einem Teil des Durchsicht-HMD oder dem Durchsicht-HMD insgesamt einstellbar ist.

8. Digitales chirurgisches Mikroskopiesystem nach Anspruch 1, wobei das Erfassen eines umfasst von:
Erfassen aus dem Bildsensor mit voller Auflösung, und
Erfassen aus dem Bildsensor durch Unterabtastung.

9. Digitales chirurgisches Mikroskopiesystem nach Anspruch 1, wobei der Bildsensor eine Pixelauflösung aufweist, die höher als eine Pixelauflösung des HMD ist.

10. Digitales chirurgisches Mikroskopiesystem nach Anspruch 1, wobei der Prozessor eine Bildgrößenänderung an der ROI vornimmt, damit sie zu der Auflösung des HMD passt.

11. Nicht endoskopisches Verfahren in der digitalen chirurgischen Mikroskopie zum Anzeigen von Vergrößerungen eines operierten Bereichs auf einem Head-Mounted-Display (HMD), wobei das Verfahren folgende Schritte umfasst:
Aufnehmen, durch eine Kamera, von Bildern eines gesamten interessierenden Blickfeldes (FOV) in dem operierten Bereich eines Patienten zur digitalen Vergrößerung des operierten Bereichs, mit Abstand zum Patienten;
Empfangen einer Eingabe, die eine Interessenregion (ROI) des operierten Bereichs angibt;
Erzeugen von ROI-Bildern, die aus den Bildern zugeschnitten werden durch eines von:
Erfassen eines Vollbildes aus einem Bildsensor und digitales Zuschneiden der ROI aus dem Vollbild; oder
Erfassen der ROI aus dem Bildsensor und nicht des Vollbildes; und
Anzeigen der ROI-Bilder.

## Revendications

1. Système de microscopie chirurgicale numérique, comprenant :
une caméra configurée pour capturer des images d'une zone opérée ;
un visio-casque (HMD) configuré pour afficher des grossissements de ladite zone opérée à un utilisateur,
en affichant des images de régions d'intérêt (ROI) découpées à partir desdites images ;
une unité de mémoire ; et
un dispositif de traitement couplé audit HMD, à ladite caméra et à ladite unité de mémoire, ledit dispositif de traitement étant configuré pour recevoir une entrée indiquant ladite ROI et configuré pour produire lesdites images de ROI soit :
en saisissant une image pleine d'un capteur d'image de ladite caméra et en découpant ladite ROI numériquement à partir de ladite image pleine ; soit
en saisissant ladite ROI dudit capteur d'image et non ladite image pleine ;
dans lequel ladite unité de mémoire est configurée pour stocker ladite image pleine ou ladite ROI ;
dans lequel ledit système de microscopie chirurgicale numérique est un système de non-endoscopie.

2. Système de microscopie chirurgicale numérique selon la revendication 1, comprenant en outre un pointeur configuré pour pointer une ligne de vue (LOS) d'utilisateur dudit utilisateur, ledit système de microscopie chirurgicale numérique étant en outre configuré pour changer un emplacement de ROI ou une taille de ROI, selon ladite LOS.

3. Système de microscopie chirurgicale numérique selon la revendication 2, dans lequel ledit pointeur est chacun d'un pointeur oculaire et d'un pointeur de tête.

4. Système de microscopie chirurgicale numérique selon la revendication 1, dans lequel ledit dispositif de traitement est configuré pour appliquer au moins un algorithme de traitement d'image ou de traitement de vidéo prédéfini auxdites images ROI.

5. Système de microscopie chirurgicale numérique selon la revendication 1, dans lequel ledit dispositif de traitement est configuré pour ajouter des marqueurs sur une image affichée par ledit HMD.

6. Système de microscopie chirurgicale numérique selon la revendication 1, dans lequel ledit HMD est un HMD transparent dont la transparence est réglable.

7. Système de microscopie chirurgicale numérique selon la revendication 6, dans lequel ladite transparence est ajustable sur une partie dudit HMD transparent ou sur tout le HMD transparent.

8. Système de microscopie chirurgicale numérique selon la revendication 1, dans lequel ladite saisie implique soit l'un de : saisir dudit capteur d'image en pleine résolution et saisir dudit capteur d'image par souséchantillonnage.

9. Système de microscopie chirurgicale numérique selon la revendication 1, dans lequel ledit capteur d'image a une résolution de pixels qui est supérieure à une résolution de pixels dudit HMD.

10. Système de microscopie chirurgicale numérique selon la revendication 1, dans lequel ledit processeur effectue un redimensionnement de l'image sur ladite ROI pour ajuster à la résolution dudit HMD.

11. Procédé non-endoscopique en microscopie chirurgicale numérique pour afficher des grossissements d'une zone opérée sur un visio-casque (HMD), le procédé comprenant :
de saisir, par une caméra, des images d'un champ entier de vue d'intérêt (FOV) dans ladite zone opérée d'un patient pour le grossissement numérique de ladite zone opérée, à une distance dudit patient ;
de recevoir une entrée indiquant une région d'intérêt (ROI) de ladite zone opérée ;
de produire des images ROI qui sont découpées à partir desdites images, soit :
en saisissant une image pleine d'un capteur d'image et en découpant ladite ROI numériquement à partir de ladite image pleine ; soit
en saisissant ladite ROI dudit capteur d'image et non de ladite image pleine ;
et
en affichant lesdites images ROI.
